# FASCICULE DE BREVET EUROPEEN

(11) **EP 4 280 935 B1**
(45) Date de publication et mention de la délivrance du brevet: **15.01.2025**
(21) Numéro de dépôt: 22701573.2
(22) Date de dépôt: 19.01.2022
(51) Int. Cl.: A61B 3/103, A61B 3/028

(54) **SYSTÈME D'ASSISTANCE À LA DÉTERMINATION D'UNE PRESCRIPTION OPTIQUE FINALE POUR UN SUJET ET PROCÉDÉ ASSOCIÉ**
SYSTEM ZUR UNTERSTÜTZUNG BEI DER BESTIMMUNG EINER ENDGÜLTIGEN OPTISCHEN VERORDNUNG FÜR EINE PERSON UND ZUGEHÖRIGES VERFAHREN
SYSTEM FOR ASSISTING IN DETERMINING A FINAL OPTICAL PRESCRIPTION FOR A SUBJECT AND ASSOCIATED METHOD

(30) Priorité: 20.01.2021 FR 2100523
(43) Date de publication de la demande: 29.11.2023
(73) Titulaire: Luneau Technology Operations, 27340 Pont de l'Arche (FR)
(72) Inventeur: LAMBERT, Frederic Albert André, 28700 HOUVILLE-LA-BRANCHE (FR)
(74) Mandataire: Lavoix
(86) Numéro de dépôt international: PCT/EP2022/051127
(87) Numéro de publication internationale: WO 2022/157195

(56) Documents cités:
- FR-A1- 3 050 922
- JP-A- H09 192 100
- KR-B1- 102 149 148

## Description

La présente invention concerne un système d'assistance à la détermination d'une prescription optique finale pour un sujet, comprenant :
- au moins un dispositif de mesure, destiné à mesurer un jeu de données de réfraction à partir des yeux du sujet ou de verres correcteurs du sujet, et
- un dispositif d'évaluation, configuré pour proposer au moins un jeu de verres correcteurs d'évaluation à un praticien, pour le sujet, chaque jeu de verres correcteurs d'évaluation correspondant à une prescription optique d'évaluation, pour la détermination, par le sujet, de la prescription optique finale, à partir de l'au moins un jeu de verres correcteurs d'évaluation proposé,
le dispositif d'évaluation déterminant l'au moins un jeu de verres correcteurs d'évaluation proposé en fonction du jeu de données de réfraction mesuré.

Dans le domaine de la prescription optique, les praticiens utilisent généralement un système comprenant plusieurs dispositifs distincts pour déterminer la prescription optique finale d'un sujet.

Ce système comprend notamment des dispositifs de mesure objective d'un paramètre physique sur des verres correcteurs d'un sujet, si le sujet porte déjà des verres, et/ou des dispositifs de mesure directe sur les yeux du sujet.

FR 3 050 922 divulgue un réfractomètre automatique objectif comprenant une réserve de verres correcteurs.

La ou les valeur(s) mesurée(s) forme(nt) généralement un ou des jeu(x) de données de réfraction permettant de déterminer approximativement la prescription optique appropriée pour le sujet.

Un tel système comprend également au moins un dispositif d'évaluation subjective, dans lequel le sujet détermine par au moins un essai de verres correcteurs d'évaluation quels sont les verres correcteurs étant les plus appropriés pour corriger sa vue tout en assurant son confort, permettant ainsi de déterminer la prescription optique finale.

En vue de limiter les essais nécessaires pour déterminer la prescription optique finale, il est d'usage d'utiliser un ou des dispositifs de mesure objective pour déterminer au moins un jeu de données de réfraction, puis d'utiliser ce jeu de données de réfraction dans le dispositif d'évaluation subjective, afin de rapidement proposer au sujet des verres correcteurs d'évaluation susceptibles d'être appropriés pour corriger sa vue.

Avec le développement des technologies informatiques, le transfert des données de réfraction depuis les dispositifs de mesure objective vers les dispositifs d'évaluation subjective a été simplifié. Il est désormais connu d'utiliser des systèmes comprenant des serveurs d'échange de données entre les dispositifs de mesure et les dispositifs d'évaluation.

De tels systèmes ne donnent toutefois pas entière satisfaction. En effet, l'installation de serveurs d'échange est complexe et coûteuse. En outre, un tel système repose sur un réseau informatique, qui, s'il vient à tomber en panne, risque d'empêcher toute prescription optique.

Un but de l'invention est alors de proposer un système d'assistance à la détermination d'une prescription optique finale pour un sujet qui soit peu complexe, peu coûteux et robuste, tout en restant pratique à utiliser pour le praticien.

A cet effet, l'invention a pour objet un système tel que précité dans lequel le dispositif de mesure comprend des moyens de production d'une image codant le jeu de données de réfraction mesuré, le dispositif d'évaluation comprenant des moyens de lecture de l'image, configurés pour obtenir le jeu de données de réfraction mesuré à partir de l'image lue.

Un système comprenant dispositif de mesure comprenant des moyens de production d'une image codant un jeu de données de réfraction d'une part et comprenant un dispositif d'évaluation comprenant des moyens de lecture de l'image d'autre part, facilite la transmission de données de réfraction depuis le dispositif de mesure vers le dispositif d'évaluation aisée, tout en assurant une robustesse de la transmission, sans la nécessité d'une infrastructure coûteuse.

Suivant d'autres aspects avantageux de l'invention, le système d'assistance à la détermination d'une prescription optique finale pour un sujet comprend une ou plusieurs des caractéristiques suivantes, prises isolément ou suivant toutes les combinaisons techniquement possibles :
- les moyens de production de l'image comprennent une imprimante, l'image étant imprimée sur une feuille ;
- l'image est un code QR ;
- l'image correspond à un message crypté correspondant au jeu de réfraction mesuré, les moyens de lecture de l'image étant configurés pour désencrypter le message crypté pour obtenir le jeu de données de réfraction mesuré ;
- le jeu de données de réfraction mesuré comprend une valeur de sphère représentatif d'une puissance de myopie ou d'hypermétropie, une valeur de cylindre représentative d'une puissance d'astigmatisme et une valeur d'axe représentatif d'un axe d'astigmatisme ;
- le dispositif d'évaluation comprend une unité de contrôle et une unité d'évaluation, l'unité de contrôle étant mobile par rapport à l'unité d'évaluation et comprenant les moyens de lecture de l'image, l'unité de contrôle étant configurée pour transmettre à l'unité d'évaluation une information caractérisant l'au moins un jeu de verre correcteur d'évaluation à proposer ;
- le dispositif de mesure est un autoréfractomètre destiné à mesurer un jeu de données de réfraction à partir des yeux du sujet, le dispositif d'évaluation étant un réfracteur ;
- le dispositif de mesure est un lensomètre destiné à mesurer un jeu de données de réfraction à partir de verres correcteur du sujet, le dispositif d'évaluation comprenant un réfracteur et un autoréfractomètre, l'autoréfractomètre étant destiné à mesurer un jeu de données de réfraction complémentaire à partir des yeux du sujet, le dispositif d'évaluation déterminant l'au moins un jeu de verres correcteurs d'évaluation proposé en fonction du jeu de données de réfraction mesuré et du jeu de réfraction complémentaire mesuré ; et
- le système comprend deux dispositifs de mesure:
   - un premier dispositif de mesure étant un lensomètre destiné à mesurer un premier jeu de données de réfraction à partir de verres correcteurs du sujet,
   - un deuxième dispositif de mesure étant un autoréfractomètre destiné à mesurer un deuxième jeu de données de réfraction à partir des yeux du sujet, le dispositif d'évaluation déterminant l'au moins un jeu de verres correcteur d'évaluation proposé en fonction du premier et du deuxième jeux de données de réfraction mesurés,
   chaque dispositif de mesure comprenant des moyens de production d'une image codant le jeu de données de réfraction que ledit dispositif de mesure a mesuré, les moyens de lecture de l'image étant configurés pour obtenir les jeux de données de réfraction mesurés à partir de chacune des images lues.

L'invention concerne en outre un procédé de détermination d'une prescription optique finale pour un sujet, comprenant les étapes suivantes :
- fourniture d'un système d'assistance à la détermination d'une prescription optique finale pour un sujet tel que précité ;
- mesure d'un jeu de données de réfraction à partir des yeux du sujet ou de verres correcteur du sujet par le dispositif de mesure ;
- production d'une image correspondant au jeu de données de réfraction mesuré par le dispositif de mesure ;
- lecture de l'image par le dispositif d'évaluation pour obtenir le jeu de données de de réfraction mesuré à partir de l'image lue; et
- proposition d'au moins un jeu de verres correcteurs d'évaluation à un praticien, pour le sujet, par le dispositif d'évaluation, en fonction du jeu de données de réfraction mesuré.

L'invention sera mieux comprise à la lecture de la description qui va suivre, donnée uniquement à titre d'exemple non limitatif et faite en se référant aux figures suivantes, dans lesquelles :
[Fig 1] la figure 1 est une représentation schématique de différents dispositifs d'un système d'assistance selon un premier mode de réalisation de l'invention ;
[Fig 2] la figure 2 est une représentation schématique de différents dispositifs d'un système d'assistance selon un deuxième mode de réalisation de l'invention ;
[Fig 3] la figure 3 est une représentation schématique de différents dispositifs d'un système d'assistance selon un troisième mode de réalisation de l'invention ;
[Fig 4] la figure 4 est une illustration d'un premier jeu de données de réfraction et d'une image correspondant au premier jeu de données de réfraction ;
[Fig 5] la figure 5 est une illustration d'un deuxième jeu de données de réfraction et d'une image correspondant au deuxième jeu de données de réfraction ;
[Fig 6] la figure 6 est un organigramme d'un procédé de détermination d'une prescription selon l'invention ; et
[Fig 7] la figure 7 est une représentation schématique d'une unité de contrôle d'un dispositif d'évaluation d'un système tel que présenté sur les figures 1 à 3.

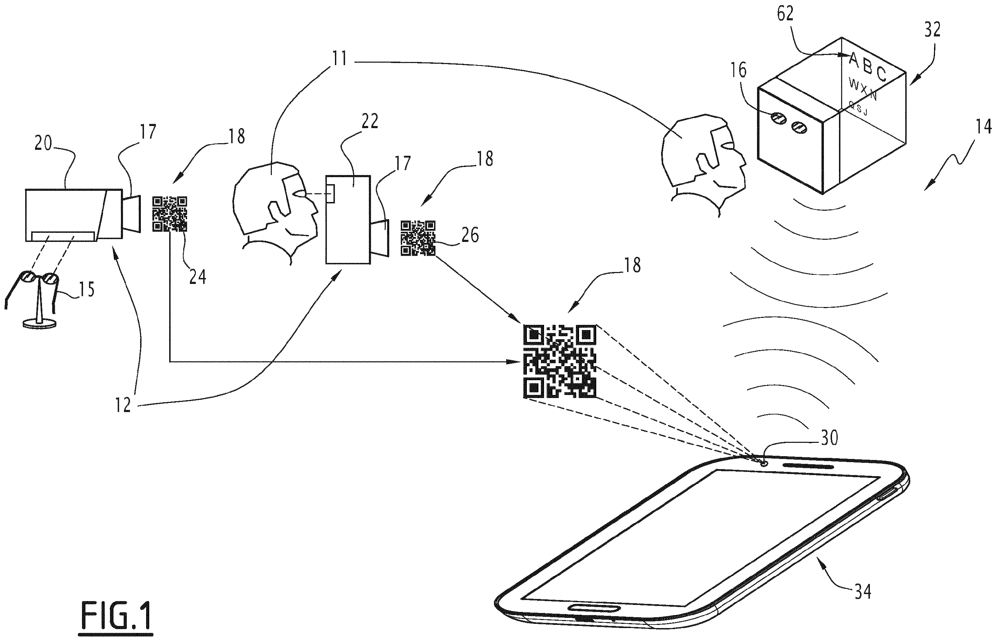

En référence aux figures 1 à 3, un système 10 d'assistance à la détermination d'une prescription optique finale pour un sujet 11 comprend au moins un dispositif de mesure 12 et un dispositif d'évaluation 14.

Le système 10 d'assistance à la détermination d'une prescription optique finale pour un sujet 11, aussi appelé dans la suite de la description système 10 d'assistance à la prescription ou système d'assistance 10, est destiné à être installé dans un local (non représenté) tel par exemple un cabinet d'ophtalmologie, un local d'opticien ou tout autre local ou ensemble de locaux propre(s) à la détermination d'une prescription optique finale pour un sujet 11.

La prescription optique finale correspond à une prescription de verres correcteurs finaux permettant de corriger la vue du sujet 11. La prescription optique finale comprend en particulier l'ensemble des informations nécessaires à la fabrication de verres correcteurs finaux configurés pour corriger la vue du sujet 11.

Le ou chaque dispositif de mesure 12 est destiné à mesurer objectivement un jeu de données de réfraction à partir des yeux du sujet 11 ou de verres correcteurs 15 du sujet. Par verres correcteurs 15 du sujet, on entend ici des verres correcteurs, existant préalablement à la détermination de la prescription optique finale, et devant par exemple être renouvelés et/ou changés.

Le jeu de données de réfraction mesuré comprend des données de réfraction représentant les propriétés optiques mesurées des yeux du sujet 11 ou des verres correcteurs 15 du sujet. Le jeu de données de réfraction mesuré forme ainsi le résultat d'une mesure objective de propriétés optiques.

Le jeu de données de réfraction mesuré comprend par exemple, pour chaque oeil ou chaque verre correcteur du sujet, une valeur de sphère, notée S, représentative d'une puissance de myopie ou d'hypermétropie du sujet dont les yeux ou les verres correcteurs ont été mesurés.

Le jeu de données de réfraction mesuré comprend par exemple, pour chaque oeil ou chaque verre correcteur du sujet, une valeur de cylindre, notée C, représentative d'une puissance d'astigmatisme du sujet dont les yeux ou les verres correcteurs ont été mesurés.

Le jeu de données de réfraction mesuré comprend par exemple, pour chaque oeil ou chaque verre correcteur du sujet, une valeur d'axe, notée A, représentative d'un axe d'astigmatisme du sujet dont les yeux ou les verres correcteurs ont été mesurés.

Le jeu de données de réfraction mesuré comprend par exemple une valeur de distance pupillaire, notée PD, représentative de la distance entre le centre des pupilles du sujet dont les yeux ou les verres ont été mesurés. Le jeu de données comprend par exemple, en alternative ou en complément, deux valeurs de demi écart pupillaire, notées, RDP ou R et LDP ou L, représentatives de l'écart entre le centre des pupilles droites, respectivement gauche, du sujet et le plan formé par le milieu du nez du sujet.

Le jeu de données de réfraction mesuré comprend par exemple un identifiant, noté ID, permettant d'associer les données de réfraction mesurées à une mesure des yeux ou des verres correcteurs 15 du sujet 11, référençant ainsi le jeu de données de réfraction mesuré.

Le dispositif de mesure 12 comprend des moyens 17 de production d'une image 18, aussi appelés par la suite moyens de production 17.

Dans une variante préférée, les moyens de production 17 comprennent un processeur d'encodage, propre à créer l'image 18 sur la base du jeu de données, et une imprimante configurée pour imprimer l'image 18. Selon cette variante, l'image 18 est par exemple imprimée sur une feuille, par exemple sur une feuille en papier et/ou une feuille autocollante. L'imprimante est par exemple une imprimante thermique.

En alternative, les moyens de production 17 comprennent un écran d'affichage configuré pour afficher l'image 18 engendrée par le processeur d'encodage.

L'image 18 code le jeu de donnés de réfraction mesuré par le dispositif de mesure 12.

L'image 18 est de préférence un code QR, aussi appelé en anglais *QR code,* abréviation du terme « *Quick Response Code ».* L'image 18 est alors une matrice de blocs noirs sur fond blanc formant un code barre bidimensionnel.

L'image 18 correspond de préférence à un message crypté correspondant au jeu de réfraction mesuré. Le message crypté ne peut alors être décrypté que par un dispositif configuré pour désencrypter le message crypté, par exemple à l'aide d'une clé de décryptage. Lorsque les moyens de production 17 de l'image 18 sont configurés pour produire une image cryptée, le dispositif d'évaluation est configuré pour désencrypter le message crypté tel que cela sera décrit par la suite.

Lorsque l'image 18 est un QR code et que l'image correspond à un message crypté, le code formé par l'image forme un QR code crypté, ou QR code sécurisé, aussi abrévié SQR code, de l'anglais « *Secured Quick Responde Code ».*

Les moyens de production 17 sont par exemple configurés pour imprimer/afficher le jeu de données de réfraction mesuré codés par l'image 18, à côté de l'image 18. En variante, les moyens de production 17 ne sont configurés que pour imprimer/afficherl'image 18.

Dans le mode de réalisation de la figure 1, le système 10 comprend deux dispositifs de mesure 12. Un premier dispositif de mesure 20 est un lensomètre, destiné à mesurer un jeu de données de réfraction à partir de verre correcteurs du sujet. Un deuxième dispositif de mesure 22 est un autoréfractomètre, destiné à mesurer un deuxième jeu de données de réfraction à partir des yeux du sujet 11.

Chaque dispositif de mesure 12 comprend alors des moyens 17 de production d'une image 18 codant le jeu de données de réfraction que ledit dispositif de mesure 12 vient mesurer.

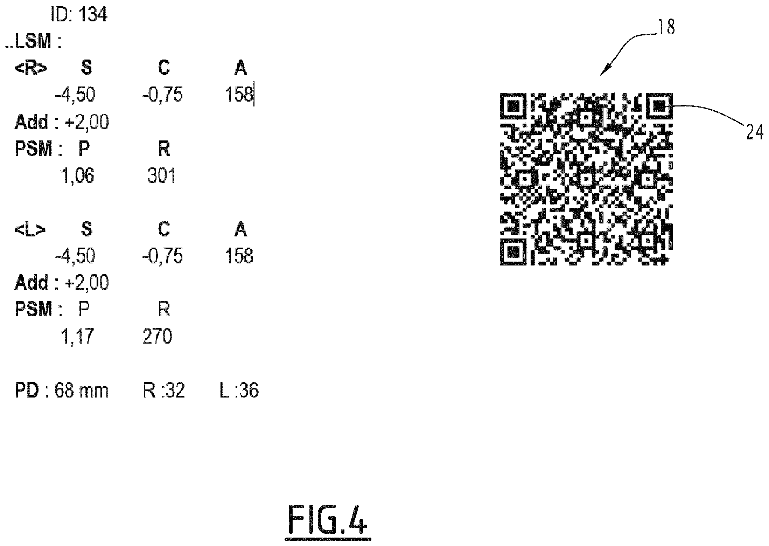

Les moyens 17 de production d'une image 18 du premier dispositif de mesure 20 sont configurés pour produire une première image 24 codant un premier jeu de données de réfraction mesuré par le premier dispositif de mesure 20. La figure 4 représente un exemple de premier jeu de données, sur la gauche de la figure, et un exemple de première image 24, codant le premier jeu de données de réfraction mesuré, sur la droite de la figure.

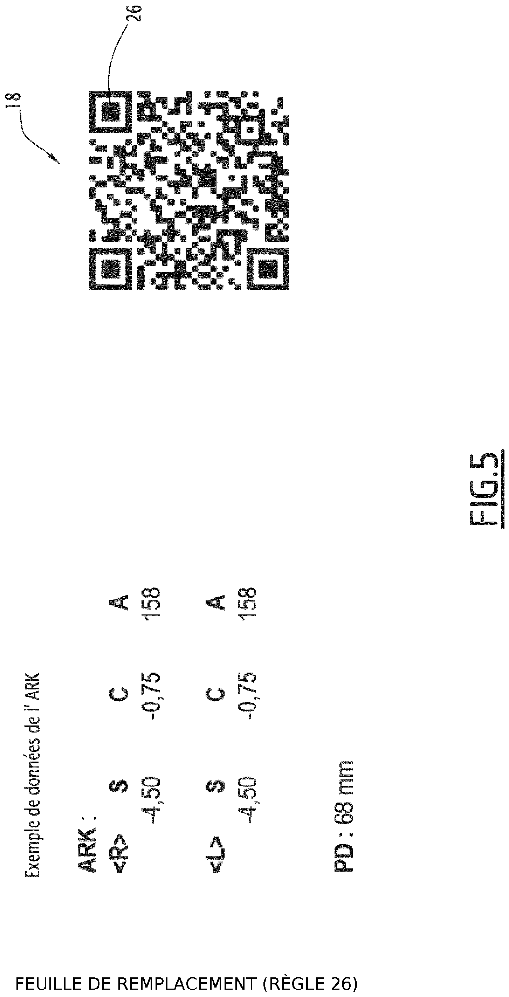

Les moyens 17 de production d'une image 18 du deuxième dispositif de mesure 22 sont configurés pour produire une deuxième image 26 codant un deuxième jeu de données de réfraction mesuré. La figure 5 représente un exemple de deuxième jeu de données, sur la gauche de la figure, et un exemple de deuxième image 26, codant le deuxième jeu de données de réfraction mesuré, sur la droite de la figure.

Le dispositif d'évaluation 14 est configuré pour proposer au moins un jeu de verres correcteurs d'évaluation 16 à un praticien (non représenté), pour le sujet 11, chaque jeu de verres correcteurs d'évaluation 16 correspondant à une prescription optique d'évaluation.

Le dispositif d'évaluation 14 est par exemple configuré pour afficher une information caractérisant les verres correcteurs d'évaluation 16 au praticien, le praticien proposant, par l'intermédiaire du dispositif d'évaluation 14, et par exemple tour à tour, les verres correcteurs d'évaluation 16 au sujet.

Le dispositif d'évaluation 14 est configuré pour permettre au sujet de déterminer une prescription optique finale, à partir de l'au moins un jeu de verres correcteurs d'évaluation 16 proposé.

Le dispositif d'évaluation 14 comprend par exemple un réfracteur, aussi connu sous la dénomination anglaise de « *phoropter* ». Dans le mode de réalisation de la figure 1, le dispositif d'évaluation est un réfracteur.

Le dispositif d'évaluation 14 comprend des moyens de lecture 30 de l'image 18.

Le dispositif d'évaluation 14 comprend par exemple une unité d'évaluation 32 et une unité de contrôle 34.

L'unité de contrôle 34 comprend par exemple les moyens de lecture 30 de l'image 18 et un module de commande 40.

Dans une variante particulière, l'unité de contrôle 34 est mobile par rapport à l'unité d'évaluation 32.

L'unité de contrôle 34 est par exemple connectée à l'unité d'évaluation 32 à l'aide d'une connexion sans fil. La connexion sans fil est une connexion d'échange de données, par exemple de type Bluetooth.

L'unité de contrôle 34 est par exemple formée par une tablette ou un smartphone.

Les moyens de lecture 30 de l'image 18 sont configurés pour lire l'image 18 et sont en particulier configurés pour obtenir le jeu de données de réfraction mesuré par le dispositif de mesure 12, à partir de l'image 18 lue.

Les moyens 30 de lecture de l'image 18 comprennent par exemple, ou sont connectés à, un capteur photographique, et sont configurés pour acquérir des données représentatives de l'image 18. Les moyens 30 de lecture de l'image 18 sont en outre configurés pour interpréter les données représentatives de l'image 18 et pour obtenir le jeu de données de réfraction à partir de ces données représentatives de l'image 18.

Lorsque l'image 18 correspond à un message crypté, les moyens de lecture 30 de l'image sont par exemple configurés pour désencrypter le message crypté pour obtenir le jeu de réfraction mesuré.

Les moyens de lecture 30 comportent par exemple un module 42 d'acquisition de l'image et un module 44 d'interprétation de l'image 18, le module d'acquisition 42 étant configuré pour obtenir les données représentatives de l'image 18 et le module d'interprétation 44 étant configuré pour interpréter les données représentatives de l'image 18.

Dans le mode de réalisation de la figure 1, selon laquelle un premier dispositif de mesure 20 produit une première image 24 correspondant à un premier jeu de données de réfraction et un deuxième dispositif de mesure 22 produit une deuxième image 26 correspondant à un deuxième jeu de données de réfraction, les moyens de lecture 30 de l'image sont configurés pour obtenir les jeux de données de réfraction mesurés à partir de chacune des images 24, 26 lues.

Le module de commande 40 est configuré pour déterminer au moins un jeu de verres correcteurs d'évaluation 16, à partir du jeu de données de réfraction mesuré lu par les moyens 30 de lecture de l'image 18. Le dispositif d'évaluation 14 détermine donc ainsi l'au moins un jeu de verres correcteurs d'évaluation 16 proposé au sujet 11 en fonction du jeu de données de réfraction mesuré.

Dans le mode de réalisation de la figure 1, le module de commande 40, et ainsi, le dispositif d'évaluation 14, détermine l'au moins un jeu de verres correcteurs d'évaluation 16 proposé au en fonction du premier et du deuxième jeux de données de réfraction mesurés.

L'unité de contrôle 34 est par exemple configurée pour déterminer une information caractérisant l'au moins un jeu de verres correcteurs d'évaluation à proposer à partir du ou des jeu(x) de données de réfraction mesuré(s). L'unité de contrôle 34 est par exemple configurée pour transmettre à l'unité d'évaluation 32, par exemple par l'intermédiaire du module de commande 40 de l'unité de contrôle 34, l'information caractérisant l'au moins un jeu de verre correcteur d'évaluation 16 à proposer.

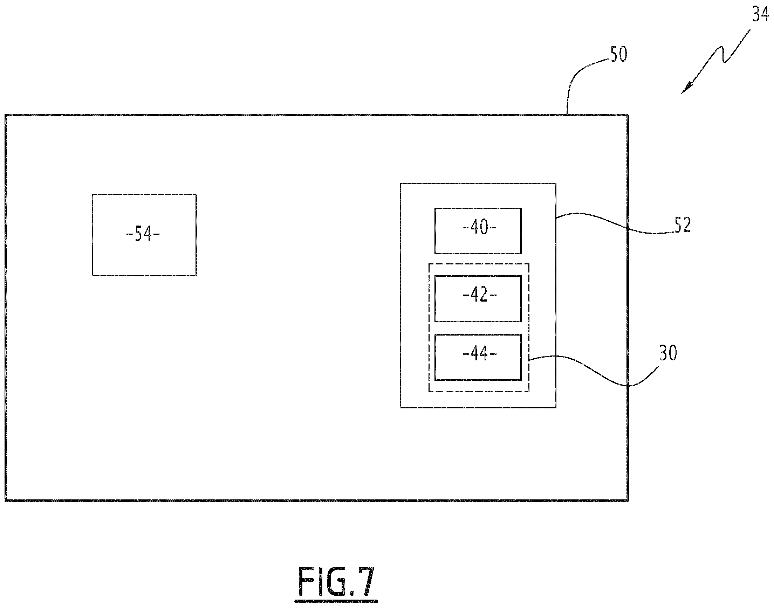

Dans un mode de réalisation particulier, tel que présenté sur la figure 7, l'unité de contrôle 34 comprend une unité de traitement d'informations 50 formée par exemple d'une mémoire 52 associée à un processeur 54.

Le module de commande 40, et de préférence le module d'acquisition 42 et le module d'interprétation 44 sont réalisé chacun sous forme d'un logiciel exécutable par le processeur 54. La mémoire 52 est alors apte à stocker un logiciel d'acquisition d'image, un logiciel de d'interprétation d'image et un logiciel de commande. Le processeur 54 de l'unité de traitement d'informations 50 est alors apte à exécuter le logiciel de commande, le logiciel d'acquisition et le logiciel d'interprétation

En variante, le module d'acquisition 42, le module d'interprétation 44 et le module de commande 40 sont réalisés chacun sous forme d'un composant logique programmable, tel qu'un FPGA (de l'anglais *Field Programmable Gate Array*)*,* ou encore sous forme d'un circuit intégré dédié, tel qu'un ASIC (de l'anglais *Application Spécifie Integrated Circuit*)*.*

Lorsque le module de commande 40, le module d'acquisition 42 et le module d'interprétation 44 sont réalisés sous forme d'un ou plusieurs logiciels, c'est-à-dire sous forme d'un programme d'ordinateur, il sont en outre apte à être enregistré sur un support, non représenté, lisible par ordinateur. Le support lisible par ordinateur est par exemple, un médium apte à mémoriser des instructions électroniques et à être couplé à un bus d'un système informatique. A titre d'exemple, le support lisible est un disque optique, un disque magnéto-optique, une mémoire ROM, une mémoire RAM, tout type de mémoire non volatile (par exemple EPROM, EEPROM, FLASH, NVRAM), une carte magnétique ou une carte optique. Sur le support lisible est alors mémorisé un programme d'ordinateur comprenant des instructions logicielles.

L'unité d'évaluation 32 comprend au moins un jeu de verres correcteurs d'évaluation 16, et comprend par exemple une région d'accueil 62 de l'au moins un jeu de verres correcteurs d'évaluation et une région d'affichage 64.

L'unité d'évaluation 32 comprend par exemple une pluralité de verres correcteurs d'évaluation, qui lorsqu'ils sont mis en place dans la région d'accueil 62, forment un jeu de verres correcteur d'évaluation 16.

L'unité d'évaluation 32 est configurée pour proposer au moins un jeu de verres correcteurs d'évaluation en fonction de l'information caractérisant l'au moins un jeu de verre correcteur d'évaluation 16 à proposer, ayant été transmise par l'unité de contrôle 34. L'unité d'évaluation 32 est ainsi configurée pour placer les verres correcteurs d'évaluation dans la région d'accueil 62, afin de former l'au moins un jeu de verres correcteurs d'évaluation, en fonction de l'information caractérisant l'au moins un jeu de verre correcteur d'évaluation 16 à proposer.

Lorsque plusieurs jeux de verres correcteurs d'évaluation 16 sont proposés, l'unité d'évaluation 32 est par exemple configurée pour proposer les jeux de verre d'évaluation les uns à la suite des autres, permettant au sujet s'installant face à la région d'accueil de tester chacun de ces jeux de verres correcteurs d'évaluation 16 successivement.

Comme illustré sur les figures 1 à 3, la région d'affichage 62, s'étend par exemple en regard de la région d'accueil de l'au moins un jeu de verres correcteurs d'évaluation, en particulier de sorte à ce qu'un sujet s'installant face à la région d'accueil observe la région d'affichage 62 au travers du jeu de verres correcteur d'évaluation 16 proposé.

La région d'affichage 62 comprend par exemple une échelle de Monoyer comme illustré sur les figures 1 à 3.

Le dispositif d'évaluation permet ainsi au sujet 11 d'évaluer de façon subjective la correction de chacun des verres correcteurs d'évaluation 16 proposés, lui permettant ainsi de déterminer les verres correcteurs d'évaluation proposés qu'il trouve les plus adéquats et lui permettant ainsi de déterminer la prescription optique finale, correspondant aux verres correcteurs d'évaluation 16 proposés qu'il trouve les plus adéquats.

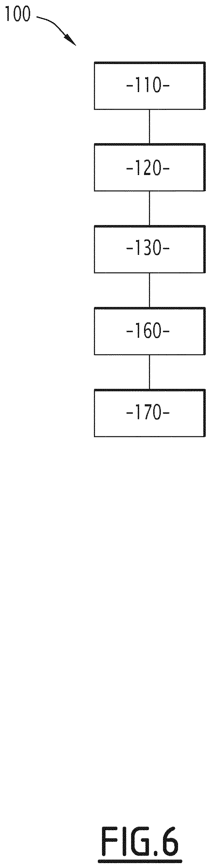

En référence à la figure 6, un procédé 100 de détermination d'une prescription optique finale pour un sujet 11 va maintenant être présenté.

Lors d'une étape de fourniture 110, un sujet 11 telque précédemment décrit se rend dans un local muni d'un système 10 d'assistance à la détermination d'une prescription optique finale.

Lors d'une étape de mesure 120 suivant l'étape de fourniture 110, un praticien active le dispositif de mesure 12 pour mesurer un jeu de données de réfraction à partir des yeux du sujet 11 ou de verres correcteurs 15 du sujet.

En particulier, en utilisant le système 10 tel que présenté sur la figure 1, un premier jeu de réfraction est mesuré par un premier dispositif de mesure 20, étant un lensomètre, à partir de verres correcteurs 15 du sujet et un deuxième jeu de réfraction est mesuré par un deuxième dispositif de mesure 22, étant un autoréfractomètre, à partir des yeux du sujet 11.

Lors d'une étape de production 130, une image 18 correspondant au jeu de réfraction mesuré par le dispositif de mesure 12 est produite, par exemple par les moyens de production 17.

En particulier, en utilisant le système 10 tel que présenté sur la figure 1, une première image 24, correspondant au premier jeu de réfraction mesuré, et une deuxième image 26, correspondant au deuxième jeu de réfraction mesuré, sont produites respectivement par les moyens de production 17 du premier 20 et du deuxième 22 dispositifs de mesure.

Lors d'une étape de lecture 160, le praticien amène l'image 18 au voisinage des moyens de lecture 30 du dispositif d'évaluation 14. Le dispositif d'évaluation 14 lit l'image 18 pour obtenir le jeu de données de réfraction mesuré à partir de l'image 18 lue.

En utilisant le système 10 tel que présenté sur la figure 1, le dispositif d'évaluation 14 lit la première image 24 pour obtenir le premier jeu de données de réfraction et lit la deuxième image 26 pour obtenir le deuxième jeu de données de réfraction. Un praticien vient par exemple déplacer la première image 24, la deuxième image 26 et/ou le dispositif d'évaluation 14, pour amener, successivement ou simultanément, la première image 24 et la deuxième image 26 en regard de moyens de lecture 30 du dispositif d'évaluation 14, afin de permettre ou d'instruire au dispositif d'évaluation 14 de lire la première image 24 et la deuxième image 26.

Lors d'une étape de proposition 170, suivant l'étape de lecture 160, le dispositif d'évaluation 14 propose au praticien au moins un jeu de verres correcteurs d'évaluation 16 pour le sujet 11 en fonction du jeu de données de réfraction mesuré.

Lorsque le dispositif d'évaluation 14 comprend une unité d'évaluation 32 et une unité de contrôle 34 distinctes, comme par dans l'exemple des figures 1 à 3, l'unité de contrôle 34 génère par exemple une information caractérisant l'au moins un jeu de verre correcteur d'évaluation 16 à proposer à partir de l'image 18. L'unité de contrôle 34 transmet, de préférence par liaison sans fil, l'information caractérisant l'au moins un jeu de verre correcteur d'évaluation 16 à proposer, à l'unité d'évaluation 32.

En particulier, en utilisant le système 10 tel que présenté sur la figure 1, le dispositif d'évaluation 14 propose au moins un jeu de verres correcteurs d'évaluation au praticien, pour le sujet 11, en fonction du premier et du deuxième jeu de réfraction mesurés. L'unité de contrôle 34 génère par exemple une information caractérisant l'au moins un jeu de verre correcteur d'évaluation 16 à proposer à partir de la première image 24 et de la deuxième image 26.

Le dispositif d'évaluation 14 permet au praticien de proposer un jeu de verres correcteur d'évaluation à un sujet, ou permet au praticien de proposer successivement des jeux de verres correcteurs, le ou les jeu(x) de verre(s) correcteurs étant proposés en fonction du ou des jeu(x) de réfraction mesuré(s).

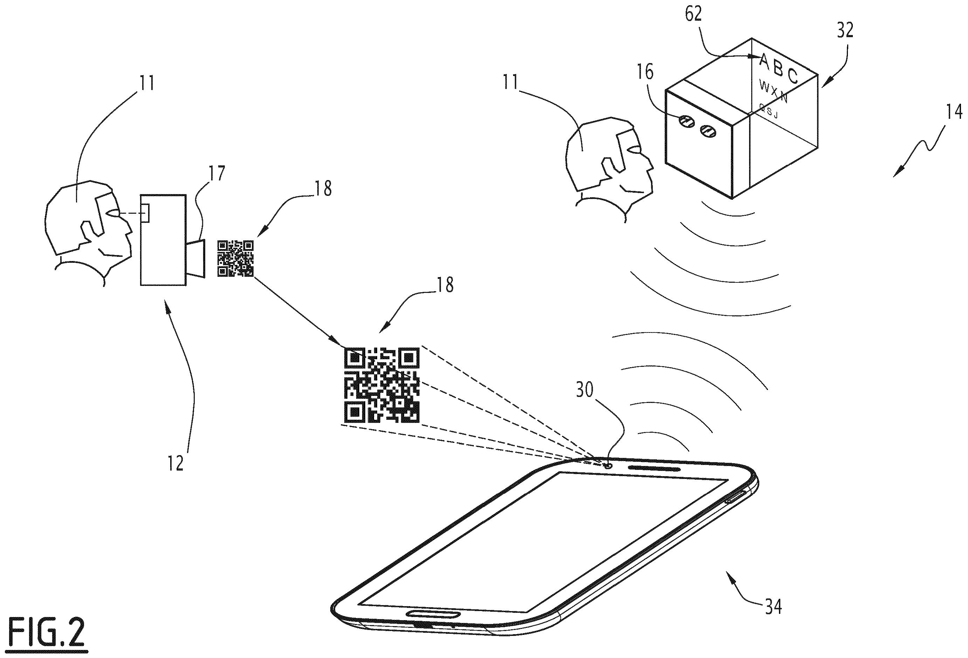

Le mode de réalisation décrit en figure 2 va maintenant être présenté en détails. Seuls les éléments différents du mode de réalisation précédemment décrit, et illustré en figure 1, sont présentés. Les éléments analogues portent les mêmes références.

Selon ce mode de réalisation, le système 10 comprend seulement un dispositif de mesure 12. Le dispositif de mesure 12 est un autoréfractomètre destiné à mesurer un jeu de données de réfraction à partir des yeux du sujet. Le dispositif de mesure 12 produit alors une image 18 codant le jeu de réfraction mesuré à partir des yeux du sujet 11. Le dispositif d'évaluation 14 est de préférence un réfracteur et propose l'au moins un jeu de verres correcteurs en fonction dudit jeu de réfraction mesuré.

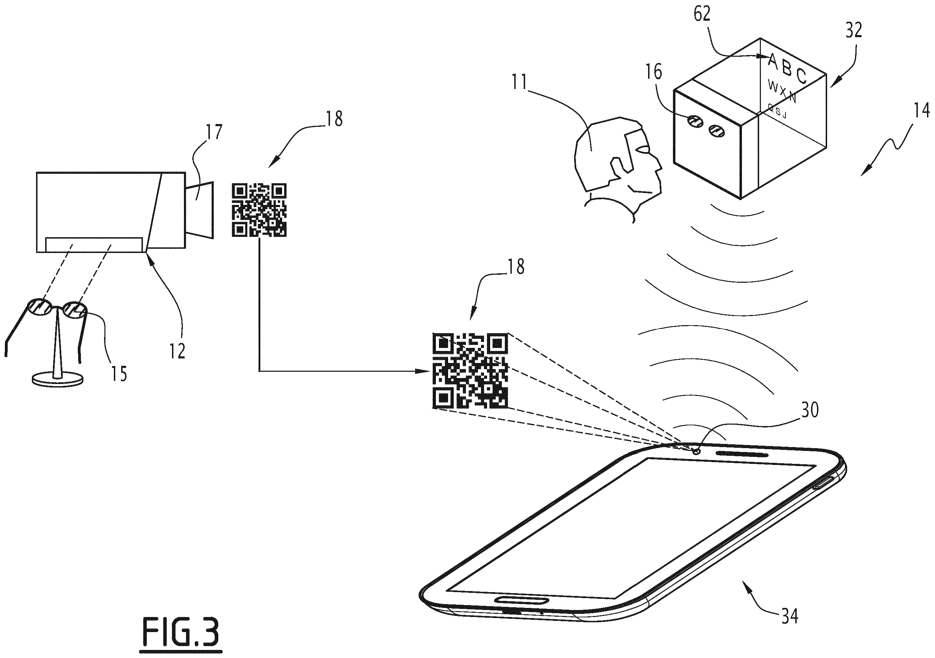

Le mode de réalisation décrit en figure 3 va maintenant être présenté en détails. Seuls les éléments différents des modes de réalisation précédemment décrits, et illustrés en figures 1 et 2, sont présentés. Les éléments analogues portent les mêmes références.

Selon ce mode de réalisation, le système comprend seulement un dispositif de mesure 12. Le dispositif de mesure 12 est un lensomètre destiné à mesure un jeu de données de réfraction à partir de verres correcteurs 15 du sujet. Le dispositif de mesure 12 produit alors un image 18 codant le jeu de réfraction mesuré à partir des verres correcteurs 15 du sujet 11.

Selon ce mode de réalisation, le dispositif d'évaluation 14 comprend un réfracteur et un autoréfractomètre. L'autoréfractomètre du dispositif d'évaluation 14 est par exemple destiné à mesurer un jeu de réfraction complémentaire à partir des yeux du sujet 11.

Le dispositif d'évaluation 14 détermine alors l'au moins un jeu de données de réfraction en fonction du jeu de données de réfraction mesuré par le lensomètre et en fonction du jeu de données de réfraction complémentaires mesuré par l'autoréfractomètre du dispositif d'évaluation.

L'utilisation d'un système 10 d'assistance dans lequel des moyens de production 17 d'une image 18 codent un jeu de données de réfraction mesuré, et des moyens de lecture 30 de l'image 18 sont configurés pour obtenir le jeu de donnée de réfraction mesurée à partir de l'image 18 lue est particulièrement avantageuse pour permettre une transmission aisée de données sans faire appel à une infrastructure informatique complexe.

L'utilisation d'une imprimante est particulièrement avantageuse pour produire simplement l'image 18, une image imprimée pouvant être conservée pendant une grande durée et pouvant facilement être déplacée entre le dispositif de mesure 12 et le dispositif d'évaluation 14.

Un code QR permet d'assurer une lecture facile de l'image 18 par le dispositif d'évaluation 32.

Un image 18 correspondant à un message crypté assure la confidentialité du jeu de données de réfraction codé par l'image, ce qui est particulièrement intéressant lorsque les données de réfraction codées par l'image 18 correspondent à des données de réfraction relatives à un sujet 11.

Un jeu de données de réfraction comprenant des valeurs de sphère, de cylindre et d'axe permet de former un jeu de données de réfraction précis et permet ainsi au dispositif d'évaluation 14 de proposer un ou des jeu(x) de verres correcteurs d'évaluation particulièrement pertinents.

Un système 10 dans lequel le dispositif de mesure est un auto réfractomètre permet d'adapter l'au moins un jeu de verres correcteurs d'évaluation 16 proposé(s) à la vue réelle du sujet 11.

Un système dans lequel le dispositif d'évaluation 14 comprend un réfracteur comprend un réfracteur et un autoréfractomètre est particulièrement intéressant puisqu'il permet de mesurer un jeu de données de réfraction à partir des yeux d'un sujet 11 et de proposer des verres correcteurs d'évaluation 16 à partir d'un unique dispositif d'évaluation 14.

Un système comprenant deux dispositifs 20, 22 de mesure est avantageux pour choisir précisément le jeu de verres correcteurs d'évaluation 16 proposé au praticien, pour le sujet 11, tout en distribuant les étapes de la détermination d'une prescription optique finale sur trois dispositifs 20, 22, 14 distincts, permettant par exemple de mieux gérer le flux de sujets 11 dont la prescription optique finale est à déterminer.

## Revendications

1. Système (10) d'assistance à la détermination d'une prescription optique finale pour un sujet (11), comprenant :
- au moins un dispositif de mesure (12), destiné à mesurer un jeu de données de réfraction à partir des yeux du sujet (11) ou de verres correcteurs (15) du sujet (11),
- un dispositif d'évaluation (14), configuré pour proposer au moins un jeu de verres correcteurs d'évaluation (16) à un praticien, pour le sujet (11), chaque jeu de verres correcteurs d'évaluation (16) correspondant à une prescription optique d'évaluation, pour la détermination, par le sujet (11), de la prescription optique finale, à partir de l'au moins un jeu de verres correcteurs d'évaluation (11) proposé,
le dispositif d'évaluation (14) déterminant l'au moins un jeu de verres correcteurs d'évaluation (16) proposé en fonction du jeu de données de réfraction mesuré, **caractérisé en ce que**, le dispositif de mesure (12) comprend des moyens de production (17) d'une image (18) codant le jeu de données de réfraction mesuré, le dispositif d'évaluation (14) comprenant des moyens de lecture (30) de l'image (18), configurés pour obtenir le jeu de données de réfraction mesuré à partir de l'image (18) lue.

2. Système (10) selon la revendication 1, dans lequel les moyens de production (17) de l'image (18) comprennent une imprimante, l'image étant imprimée sur une feuille.

3. Système (10) selon la revendication 1 ou 2, dans lequel l'image (18) est un code QR.

4. Système (10) selon l'une quelconque des revendications précédentes, dans lequel l'image (18) correspond à un message crypté correspondant au jeu de réfraction mesuré, les moyens de lecture (30) de l'image (18) étant configurés pour désencrypter le message crypté pour obtenir le jeu de données de réfraction mesuré.

5. Système (10) selon l'une quelconque des revendications précédentes, dans lequel le jeu de données de réfraction mesuré comprend une valeur de sphère représentatif d'une puissance de myopie ou d'hypermétropie, une valeur de cylindre représentative d'une puissance d'astigmatisme et une valeur d'axe représentatif d'un axe d'astigmatisme.

6. Système (10) selon l'une quelconque des revendications précédentes, dans lequel le dispositif d'évaluation (14) comprend une unité de contrôle (34) et une unité d'évaluation (32), l'unité de contrôle (34) étant mobile par rapport à l'unité d'évaluation (32) et comprenant les moyens de lecture de l'image (30), l'unité de contrôle (34) étant configurée pour transmettre à l'unité d'évaluation (32) une information caractérisant l'au moins un jeu de verre correcteur d'évaluation (16) à proposer.

7. Système (10) selon l'une quelconque des revendications 1 à 6, dans lequel le dispositif de mesure (12) est un autoréfractomètre destiné à mesurer un jeu de données de réfraction à partir des yeux du sujet, le dispositif d'évaluation (14) étant un réfracteur.

8. Système (10) selon l'une quelconque des revendications 1 à 6 dans lequel le dispositif de mesure (12) est un lensomètre destiné à mesurer un jeu de données de réfraction à partir de verres correcteur (15) du sujet, le dispositif d'évaluation (14) comprenant un réfracteur et un autoréfractomètre, l'autoréfractomètre étant destiné à mesurer un jeu de données de réfraction complémentaire à partir des yeux du sujet (11), le dispositif d'évaluation (14) déterminant l'au moins un jeu de verres correcteurs d'évaluation (16) proposé en fonction du jeu de données de réfraction mesuré et du jeu de réfraction complémentaire mesuré.

9. Système (10) selon l'une quelconque des revendication 1 à 6, dans lequel le système comprend deux dispositifs de mesure (12) :
- un premier dispositif de mesure (20) étant un lensomètre destiné à mesurer un premier jeu de données de réfraction à partir de verres correcteurs (15) du sujet (11),
- un deuxième dispositif (22) de mesure étant un autoréfractomètre destiné à mesurer un deuxième jeu de données de réfraction à partir des yeux du sujet (11),
le dispositif d'évaluation (14) déterminant l'au moins un jeu de verres correcteur d'évaluation (16) proposé en fonction du premier et du deuxième jeux de données de réfraction mesurés,
chaque dispositif de mesure (20, 22) comprenant des moyens de production (17) d'une image (18) codant le jeu de données de réfraction que ledit dispositif de mesure (20, 22) a mesuré, les moyens de lecture (30) de l'image (18) étant configurés pour obtenir les jeux de données de réfraction mesurés à partir de chacune des images (18) lues.

10. Procédé (100) de détermination d'une prescription optique finale pour un sujet (11), comprenant les étapes suivantes :
- fourniture (110) d'un système (10) d'assistance à la détermination d'une prescription optique finale pour un sujet (11) selon l'une quelconque des revendications 1 à 9 ;
- mesure (120) d'un jeu de données de réfraction à partir des yeux du sujet (11) ou de verres correcteur (15) du sujet (11) par le dispositif de mesure (12) ;
- production (130) d'une image (18) correspondant au jeu de données de réfraction mesuré par le dispositif de mesure (12) ;
- lecture (160) de l'image par le dispositif d'évaluation (14) pour obtenir le jeu de données de de réfraction mesuré à partir de l'image (18) lue; et
- proposition (170) d'au moins un jeu de verres correcteurs d'évaluation (16) à un praticien, pour le sujet (11), par le dispositif d'évaluation (14), en fonction du jeu de données de réfraction mesuré.

## Patentansprüche

1. System (10) zur Unterstützung der Bestimmung einer endgültigen optischen Verordnung für ein Subjekt (11), umfassend:
- mindestens eine Messvorrichtung (12), die zum Messen eines Refraktionsdatensatzes anhand der Augen des Subjekts (11) oder Korrekturgläser (15) des Subjekts (11) bestimmt ist,
- eine Bewertungsvorrichtung (14), die konfiguriert ist, um einem praktizierenden Arzt mindestens einen Satz von Bewertungskorrekturgläsern (16) für das Subjekt (11) vorzuschlagen, wobei jeder Satz von Bewertungskorrekturgläsern (16) einer optischen Bewertungsverordnung entspricht, um die endgültige optische Verordnung aus dem mindestens einen vorgeschlagenen Satz von Bewertungskorrekturgläsern (11) durch das Subjekt (11) zu bestimmen,
wobei die Bewertungsvorrichtung (14) den mindestens einen Satz von Bewertungskorrekturgläsern (16) bestimmt, der abhängig von dem gemessenen Refraktionsdatensatz vorgeschlagen wird, **dadurch gekennzeichnet, dass** die Messvorrichtung (12) Erzeugungseinrichtungen (17) eines Bilds (18) umfasst, das den gemessenen Refraktionsdatensatz kodiert, die Bewertungsvorrichtung (14) umfassend Leseeinrichtungen (30) des Bilds (18), die konfiguriert sind, um den Refraktionsdatensatz aus dem gelesenen Bild (18) zu erlangen.

2. System (10) nach Anspruch 1, bei dem die Erzeugungseinrichtungen (17) des Bilds (18) einen Drucker umfassen, wobei das Bild auf ein Blatt gedruckt wird.

3. System (10) nach Anspruch 1 oder 2, wobei das Bild (18) ein QR-Code ist.

4. System (10) nach einem der vorherigen Ansprüche, wobei das Bild (18) einer verschlüsselten Nachricht entspricht, die dem gemessenen Refraktionssatz entspricht, wobei die Leseeinrichtungen (30) des Bilds (18) konfiguriert sind, um die verschlüsselte Nachricht zu entschlüsseln, um den gemessenen Refraktionsdatensatz zu erlangen.

5. System (10) nach einem der vorherigen Ansprüche, wobei der gemessene Refraktionsdatensatz einen Kugelwert, der repräsentativ für eine Stärke von Kurzsichtigkeit oder Weitsichtigkeit ist, einen Zylinderwert, der repräsentativ für eine Stärke von Astigmatismus ist, und einen Achsenwert, der repräsentativ für eine Achse von Astigmatismus ist, umfasst.

6. System (10) nach einem der vorherigen Ansprüche, wobei die Bewertungsvorrichtung (14) eine Steuereinheit (34) und eine Bewertungseinheit (32) umfasst, wobei die Steuereinheit (34) in Bezug auf die Bewertungseinheit (32) beweglich ist und die Leseeinrichtungen des Bilds (30) umfasst, die Steuereinheit (34) konfiguriert ist, um Informationen an die Bewertungseinheit (32) zu übertragen, die den mindestens einen Satz von vorzuschlagenden Bewertungskorrekturgläsern (16) charakterisiert.

7. System (10) nach einem der Ansprüche 1 bis 6, wobei die Messvorrichtung (12) ein Autorefraktometer zum Messen eines Refraktionsdatensatzes anhand der Augen des Subjekts ist, wobei die Bewertungsvorrichtung (14) ein Refraktor ist.

8. System (10) nach einem der Ansprüche 1 bis 6, wobei die Messvorrichtung (12) ein Lensmeter zum Messen eines Refraktionsdatensatzes von Korrekturgläsern (15) des Subjekts ist, die Bewertungsvorrichtung (14) umfassend einen Refraktor und ein Autorefraktometer, wobei das Autorefraktometer dazu bestimmt ist, einen komplementären Refraktionsdatensatz anhand der Augen des Subjekts (11) zu messen, die Bewertungsvorrichtung (14) den mindestens einen Satz von Bewertungskorrekturgläsern (16) bestimmt, der basierend auf dem gemessenen Refraktionsdatensatz und dem gemessenen zusätzlichen Refraktionsdatensatz vorgeschlagen wird.

9. System (10) nach einem der Ansprüche 1 bis 6, wobei das System zwei Messvorrichtungen (12) umfasst:
- eine erste Messvorrichtung (20), die ein Lensmeter ist, das dazu bestimmt ist, um anhand Korrekturgläser (15) des Subjekts (11) einen ersten Refraktionsdatensatz zu messen,
- eine zweite Messvorrichtung (22), die ein Autorefraktometer ist, das dazu bestimmt ist, um anhand der Augen des Subjekts (11) einen zweiten Refraktionsdatensatz zu messen,
wobei die Bewertungsvorrichtung (14) den mindestens einen vorgeschlagenen Satz von Bewertungskorrekturgläsern (16) basierend auf dem ersten und dem gemessenen Refraktionsdatensatz bestimmt,
jede Messvorrichtung (20, 22) umfassend Erzeugungseinrichtungen (17) eines Bilds (18), das den Refraktionsdatensatz kodiert, den die Messvorrichtung (20, 22) gemessen hat, wobei die Leseeinrichtungen (30) des Bilds (18) konfiguriert sind, um die gemessenen Refraktionsdatensätze anhand jedes der gelesenen Bilder (18) zu erlangen.

10. Verfahren (100) zum Bestimmen einer endgültigen optischen Verordnung für ein Subjekt (11), umfassend die folgenden Schritte:
- Bereitstellen (110) eines Systems (10) zur Unterstützung der Bestimmung einer endgültigen optischen Verordnung für ein Subjekt (11) nach einem der Ansprüche 1 bis 9;
- Messen (120) eines Refraktionsdatensatzes anhand der Augen des Subjekts (11) oder Korrekturgläser (15) des Subjekts (11) durch die Messvorrichtung (12);
- Erzeugen (130) eines Bilds (18), das dem Refraktionsdatensatz entspricht, der von der Messvorrichtung (12) gemessen wird;
- Lesen (160) des Bilds durch die Bewertungsvorrichtung (14), um den Refraktionsdatensatz anhand des gelesenen Bilds (18) zu erlangen; und
- Vorschlagen (170), einem praktizierenden Arzt, mindestens eines Satzes von Bewertungskorrekturgläsern (16) für das Subjekt (11) durch die Bewertungsvorrichtung (14), basierend auf dem gemessenen Refraktionsdatensatz.

## Claims

1. A system (10) for assisting in determining a final optical prescription for a subject (11), comprising :
- at least one measurement device (12), intended for measuring a set of refraction data from the eyes of the subject (11) or corrective lenses (15) of the subject (11),
- an evaluation device (14), configured to propose at least one set of evaluation corrective lenses (16) to a practitioner, for the subject (11), each set of evaluation corrective lenses (16) corresponding to an evaluation optical prescription, for the determination, by the subject (11), of the final optical prescription, from the at least one proposed set of evaluation corrective lenses (11),
the evaluation device (14) determining the at least one proposed set of evaluation corrective lenses (16) as a function of the measured set of refraction data,
**characterized in that**, the measurement device (12) comprises the means (17) for producing an image (18) encoding the set of measured refraction data, the evaluation device (14) comprising the means (30) for reading the image (18), configured to obtain the set of measured refraction data from the read image (18).

2. The system (10) according to claim 1, wherein the means (17) for producing the image (18) comprises a printer, the image being printed on a sheet.

3. The system (10) according to claim 1 or 2, wherein the image (18) is a QR code.

4. The system (10) according to any one of the preceding claims, wherein the image (18) corresponds to an encoded message corresponding to the set of measured refraction data, the means (30) for reading the image (18) being configured to decode the encoded message to obtain the set of measured refraction data.

5. The system (10) according to any of the preceding claims, wherein the set of measured refraction data comprises a sphere value representative of a myopia or hyperopia power, a cylinder value representative of an astigmatism power and an axis value representative of an astigmatism axis.

6. The system (10) according to any one of the preceding claims, wherein the evaluation device (14) comprises a control unit (34) and an evaluation unit (32), the control unit (34) being movable relative to the evaluation unit (32) and comprising means of reading the image (30), the control unit (34) being configured to transmit to the evaluation unit (32) information characterizing the at least one set of evaluation corrective lenses (16) to be proposed.

7. The system (10) according to any one of claims 1 to 6, wherein the measurement device (12) is an autorefractometer for measuring a set of refraction data from the eyes of the subject, the evaluation device (14) being a refractor.

8. The system (10) according to any one of claims 1 to 6 wherein the measurement device (12) is a lensometer for measuring a set of refraction data from the corrective lenses (15) of the subject, the evaluation device (14) comprising a refractor and an autorefractometer, the autorefractometer being intended to measure a complementary set of refraction data from the eyes of the subject (11), the evaluation device (14) determining the at least one proposed set of evaluation corrective lenses (16) as a function of the set of measured refraction data and the complementary set of measured refraction data.

9. The system (10) according to any one of claims 1 to 6, wherein the system comprises two measurement devices (12):
- a first measurement device (20) being a lensometer intended to measure a first set of refraction data from the corrective lenses (15) of the subject (11),
- a second measurement device (22) being an autorefractometer for measuring a second set of refraction data from the eyes of the subject (11),
the evaluation device (14) determining the at least one proposed set of evaluation corrective lenses (16) as a function of the first and second sets of measured refraction data,
each measurement device (20, 22) comprising the means (17) for producing an image (18) encoding the set of refraction data that said measurement device (20, 22) has measured, the means for reading (30) the image (18) being configured to obtain the sets of measured refraction data from each of the images (18) read.

10. A method (100) of determining a final optical prescription for a subject (11), comprising the following steps:
- providing (110) a system (10) for assisting in determining a final optical prescription for a subject (11) according to any one of claims 1 to 9;
- measuring (120) a set of refraction data from the eyes of the subject (11) or corrective lenses (15) of the subject (11) by the measurement device (12);
- generating (130) an image (18) corresponding to the set of refraction data measured by the measurement device (12);
- reading (160) the image by the evaluation device (14) to obtain the set of measured refraction data from the read image (18); and
- proposing (170) at least one set of evaluation corrective lenses (16) to a practitioner, for the subject (11), by the evaluation device (14), according to the set of measured refraction data.
